Europäisches Patentamt

European Patent Office

Office européen des brevets

(19)

(11) Veröffentlichungsnummer : **0 074 037**
**B1**

# (12) EUROPÄISCHE PATENTSCHRIFT

(45) Veröffentlichungstag der Patentschrift :
16.04.86

(21) Anmeldenummer : 82107905.0

(22) Anmeldetag : 27.08.82

(51) Int. Cl.⁴ : **G 01 L  9/12, A 61 B  5/00**

(54) **Drucksensor, insbesondere zur Messung des intrakraniellen Druckes.**

(30) Priorität : 08.09.81 DE 3135511

(43) Veröffentlichungstag der Anmeldung :
16.03.83 Patentblatt 83/11

(45) Bekanntmachung des Hinweises auf die Patenterteilung : 16.04.86 Patentblatt 86/16

(84) Benannte Vertragsstaaten :
AT BE CH DE FR GB LI NL SE

(56) Entgegenhaltungen :
US-A- 3 968 694
US-A- 4 149 423
US-A- 4 186 749
US-A- 4 237 900
US-A- 4 265 252
FUNKSCHAU, Heft 10, 1961, Seiten 261-263, München, DE. R. DIEPKE: "Wendel-Topfkreise fpr Kreisgüten über 300"
Die Akte enthält technische Angaben, die nach dem
Eingang der Anmeldung eingereicht wurden und die
nicht in dieser Patentschrift enthalten sind.

(73) Patentinhaber : Fraunhofer-Gesellschaft zur Förderung der angewandten Forschung e.V.
Leonrodstrasse 54
D-8000 München 19 (DE)

(72) Erfinder : Koschke, Peter
Dettendorf Nr. 3
D-8201 Bad Feilnbach (DE)
Erfinder : Novak, Pavel
Görresstrasse 2
D-8000 München 40 (DE)

(74) Vertreter : Czybulka, Uwe, Dipl.-Phys et al
Patentanwälte Haft, Berngruber, Czybulka
Hans-Sachs-Strasse 5
D-8000 München 5 (DE)

## Beschreibung

Die Erfindung betrifft einen Drucksensor nach dem Oberbegriff des Anspruchs 1.

Solche Drucksensoren sind bekannt, der Schwingkreis wird von einem Oszillator getrieben un seine Eigenfrequenz, die normalerweise im MHz-Bereich liegt, schwankt in Abhängigkeit von der veränderlichen Kapazität, so daß die Frequenz-Schwankung des Oszillatorsignals mit Hilfe eines Frequenz-Detektors in eine entsprechend schwankende Gleichspannung umgewandelt werden kann. Oblicherweise wird der Frequenzdetektor für einen solchen Drucksensor justierbar ausgebildet, so daß er auf die Trägerfrequenz des Sensor-Oszillatorsystems eingestellt werden kann, beispielsweise in der Weise, daß er keine Spannung abgibt, wenn der Sensor keinem Druck ausgesetzt ist. (VDI-Bericht Nr. 93 « Verfahren der elektrischen Druckmessung und ihre Anwendung » Düsseldorf, 1966, S. 44).

Diese bekannten Drucksensoren sind nicht für alle Anwendungsfälle geeignet, weil ihre Abmessungen zu groß sind, sie über Kabel mit den übrigen Komponenten der Anlage verbunden sind, und sie keine ausreichende Langzeitstabilität aufweisen.

Es sind ferner kapazitive Druckaufnehmer bekannt, bei denen eine dünne, gespannte Edelstahlmembran mit einem Edelstahlgehäuse verschweißt ist und mit einer isolierten Elektrode, die dicht unter der Membarn angeornet ist, eine variable Kapazität bildet. Dieser bekannte Druckaufnehmer weist eine Elektronik mit Pulsbreitenmodulation mit einer Mittenfrequenz von ca. 100 kHz auf, wobei eine Digitalschaltung die druckabhängigen Kapazitätsänderungen in ein Gleichspannungssignal umsetzt (Serie 237 der Firma Setra Systems Inc.). Die Stabilität dieser Druckaufnehmer, und insbesondere die Temperaturabhängigkeit, reicht jedoch nicht für alle Anwendungsfälle aus, für viele sind die Abmessungen bei weitem zu groß, vor allem wegen der aufwendigen Elektronik, die in unmittelbarer Nähe der variablen Kapazität angeordnet ist, um das Gleichspannungs-Meßsignal zu erhalten, und die erforderliche Kabelverbindung mit den übrigen Komponenten der ganzen Anlage ist in einer Reihe von Fällen ebenfalls unzulässig.

Es sind ferner piezoresistive Halbleiter-Druckaufnehmer bekannt, die in kleinen Abmessungen (etwa 2 mm Durchmesser und 6 mm Länge) angeboten werden (EPI-080 der Firma Entran Devices, Inc.), aber wegen ihrer unzureichenden Stabilität und ihrer Temperarabhängigkeit aufwendige Kalibriermaßnahmen erfordern (M. Gaab, « Die Registrierung des intrakraniellen Druckés » Habilitationsschrift, Würzburg 1980, S. 45/46), die eine körperliche Verbindung zu anderen Komponenten des Systems zumindest zeitweise notwendig machen.

Es ist ferner bereits bekannt geworden, einen Halbleiter-Drucksensor in eine drahtlos übertragende Sonde einzusetzen, zusammen mit einem Temperatursensor, mit den Signalen beider Sensoren einen in der Sonde befindlichen Telemetrie-Sender zu modulieren und die Temperaturdrift des Drucksensors rechnerisch zu eliminieren, wobei die Sonde drahtlos mit Energie versorgt wurde (Medical & Biological Engineering & Computing, Januar 1979, S. 81-86). Nullpunktdrift und Eichung des Drucksensors können hierbei aber nicht erfaßt und erst recht nicht nachgestellt werden ; überdies gehen die Stabilitäten sowohl des Sender-oszillators als auch die des Empfänger-Oszillators in die Messung ein ; jeder dieser Mängel allein verbietet schon eine Langzeit-Messung, auch wenn im Zusammenhang mit der Bekanntgabe eine längere Betriebszeit als möglich diskutiert wird. Allen bekannten Drucksensoren ist darüberhinaus gemeinsam, daß der Herstellungsaufwand hoch ist.

Aus der US-A-4 186 749 ist ein Drucksensor zur Messung des intrakraniellen Druckes bekannt, der in einem Sensorgehäuse einen LC-Schwingkreis, eine zugeordnete Elektronik sowie eine Telemetrieeinrichtung aufweist. Die Stromversorgung für diesen Drucksensor erfolgt induktiv mit Hilfe eines Leistungsoszillators, dessen Energie von einer Induktionsspule innerhalb des Drucksensors aufgenommen und weitergeleitet wird. Die Ausgangssignale des sensorinternen Oszillators werden über eine weitere Induktionsspule nach außen zu einer extrakorporalen getrennten Empfängerbaueinheit gesendet und dort induktiv aufgenommen.

Dieser bekannte Drucksensor ist aus verschiedenen Komponenten zusammengesetzt, die sehr genau zueinander geometrisch justiert werden müssen. Wesentlich für die Güte des Drucksensors, d. h. für eine reproduzierbare Meßgenauigkeit auch über lange Meßzeiten, sind die Frequenz- und Temperaturstabilität. Der Drucksensor muß hierzu einfach justierbar sein, außerdem müssen Nullpunktdriften und interne Beeinflussungen der Funktionen einzelner Komponenten vermieden werden. Aufgrund des komplizierten Aufbaus des Drucksensors ist jedoch gerade die Justierung der einzelnen Komponenten zueinander bei den notwendig kleinen Dimensionen schwierig.

Aus der US-A-4 237 900 ist ein Drucksensor bekannt, der allerdings nicht für die Messung des intrakraniellen Drucks verwendet wird. Dieser Drucksensor weist einen Schwingkreis auf, der als Topfschwingkreis mit einer variablen Kapazität 1 und einer festen Induktivität ausgebildet ist. Die Kapazität und die Induktivität sind in einem Becher angeordnet. Der Drucksensor wird von außen induktiv mit Energie versorgt, die Oszillatorausgangssignale werden ebenfalls induktiv nach außen übertragen.

Auch dieser Drucksensor weist Mängel hinsichtlich der Frequenz- und Temperaturstabilität auf. Die Stabilitäten sowohl des Sender-Oszillators als auch die des Empfänger-Oszillators gehen in die Messung ein, eine gegenseitige Störung des LC-Schwingkreises und der innerhalb des Sensorgehäuses angeordneten Elektronik ist nicht auszuschließen. Zwar werden bei diesem bekannten Drucksensor die Signale elektronisch digital aufbereitet, um auf diese Weise eine bessere Justierbarkeit und Stabilität zu erreichen. Diese Schaltung ist jedoch recht aufwendig. Ferner ist zu berücksichtigen, daß die Induktivität durch unterschiedliche Belastungen und Temperaturen nicht als konstant zu werten ist. Bei den geringen Ausmaßen des Drucksensors können schon geringfügige geometrische Änderungen der Induktivität negativ in die Meßergebnisse eingehen.

Aufgabe der Erfindung demgegenüber ist es, einen Drucksensor verfügbar zu machen, der klein ist, geringen Herstellungsaufwand erfordert, hohe Stabilität und Temperaturunabhängigkeit aufweist und ohne körperliche Verbindung zum Gesamtsystem (Kabel, Druckschlauch) einsetzbar ist. Solche Drucksensoren werden überall da benötigt, wo der Meß- oder Druckraum unabhängig vom Gesamt-Meßsystem bewegbar bleiben soll oder muß, aber auch dort, wo an schlecht zugänglichen Stellen der Druck gemessen werden soll. Als Beispiel wird im folgenden die Messung des intrakraniellen Druckes zugrundegelegt ; eine andere Anwendung ist beispielsweise die Überwachung des Luftdruckes in Kfz-Reifen. Es sei ausdrücklich darauf hingewiesen, daß es bisher keinen Druckmesser gibt, der alle gestellten Forderungen gleichzeitig zu erfüllen vermag — so war es bisher beispielsweise nicht möglich, Langzeit-Stabilität bei Telemetrie-Anlagen zu errreichen, und zwar sowohl wegen der fehlenden Nacheichungsmöglichkeit als auch wegen der unvermeidlichen, unterschiedlichen Alterung der beiden beteiligten Oszillatoren, oder ausreichend kleine Abmessungen (Durchmesser kleiner als 7 mm, weil größere Löcher im Schädelknochen nicht wieder zuwachsen) mit der notwendigen Präzision bei einem Aufwand zu erzielen, der es erlaubt, den Sensor als Einmal-Gerät einzusetzen, was bei implantierten Sensoren wenigstens im Routinebetrieb wegen mechanischer Beschädigungen praktisch notwendig ist.

Ausgehend von dem Drucksensor der eingangs genannten Art wird die gestellte Aufgabe erfindungsgemäß durch die im kennzeichnenden Teil des Anspruchs 1 aufgeführten Merkmale gelöst.

Ein Drucksensor gemäß der Erfindung zeichnet sich durch eine einfache Konstruktion aus, bei der die für Langzeitmessungen erforderliche Frequenz- und Temperaturstabilität gegeben ist. Der Schwingkreis aus veränderlicher Kapazität und fester Induktivität wird als integrierte Baueinheit in Form eines Quasi-Wendeltopfkreises ausgebildet, bei dem der wendelförmige Innenleiter auf eine Keramik aufgebracht, vorzugsweise aufgebrannt ist, demnach im Gegensatz zu an sich bekannten Wendeltopfkreisen (vgl. z. B. Funkschau 1961, Heft 10, Seite 261) keine freie Wendel ist. Trotz des hohen Anteiles an Keramik, d. h. eines eigentlich nicht gewünschten Anteiles eines Dielektrikums, weist der so ausgebildete Schwingkreis eine hohe Güte auf. Durch diese Anordnung des wendelförmigen Innenleiters auf der Keramik können die Temperaturdrift praktisch eliminiert und die Frequenzstabilität hoch gehalten werden.

In das Sensorgehäuse ist auch die Oszillatorschaltung, die Stromversorgung und die Einrichtung zur drahtlosen Fernübertragung der Oszillatorsignale zu einer getrennten Baueinheit integriert. Diese Schaltungsteile werden auf der Rückseite, d. h. der der Keramik mit dem wendelförmigen Innenleiter abgewandten Seite des becherförmigen Sensorgehäuses angeordnet.

Eine besonders gute Temperturunabhängigkeit und Stabilität wird bei einer Ausbildung der Erfindung gemäß Anspruch 2 und insbesondere Anspruch 4 erreicht. Keramik ist bekanntlich gegen praktisch alle infrage kommenden Einflüsse, insbesondere Alter und Temperatur, weitgehend unempfindlich und bei gleichen Temperaturausdehnungskoeffizienten von Spulenkörper und Becher wird auch eine mögliche Temperaturabhängigkeit des Topfkreises beseitigt. Die elektrische Leitfähigkeit der infrage kommenden Werkstoffe würde die Güte des Wendeltopfkreises beeinträchtigen, was durch die Beschichtung der Innenfläche des Bechers mit einem elektrisch gut leitenden Material, insbesondere Silber vermieden wird. Auf diese Weise ist eine Verbesserung der Temperaturunabhängigkeit um wenigstens den Faktor 20 gegenüber bekannten kapazitiven Druckaufnehmer zu erreichen und eine Langzeitstabilität in der Größenordnung von $10^{-5}$ und besser. Als Werkstoff für den Becher kommt neben Keramik vor allem Edelstahl in geeigneter Legierung in Frage.

Zur Verstärkung des Meßeffektes werden die Maßnahmen nach Anspruch 3 angewandt ; dabei ergibt sich noch der Vorteil, daß gleichzeitig die Kapazität vergrößert wird und damit, wie bei Wendeltopfkreisen bekannt, bei gleichbleibendem Durchmesser die Länge des Topfkreises, d. h. des zylindrischen Bechers, verkürzt werden kann.

Wenn der Oszillator des Drucksensors gemäß Anspruch 6 getaktet wird, reduziert sich der Energiebedarf des Oszillators entsprechend dem Tastverhältnis der Taktung, so daß auch die Stromversorgung des Oszillators verkleinert werden kann, was sowohl für eine Batterie, z. B. eine Knopfzelle, als auch eine bekannte drahtlose Stromeinspeisung gilt.

Für die drahtlose Übertragung des Oszillatorsignales wird bei dem Drucksensor gemäß der Erfindung kein getrennter Sender benötigt ; vielmehr ist für die Telemetrie-Einrichtung lediglich eine Antenne notwendig. Bei den angestrebten kleinen Abmessungen des Drucksensors ist der Wirkungsgrad einer Antenne natürlich beschränkt, so daß gerade in Verbindung mit den zu Verfügung stehenden geringen Leistungen, das abgestrahlte Signal schwach und die Übertragung gegen Störung empfindlich ist. In Anwendungsfällen, in denen mit stärkeren Störungen zu rechnen ist, wird deshalb zweckmäßigerweise der Drucksensor gemäß Anspruch 8 ausgebildet, d. h. an den Oszillator wird ein Frequenzteiler angeschlossen, der eine im Infrarot arbeitende Leuchtdiode speist. Der Frequenzteiler wird benötigt, um mit einer Leuchtdiode mit geringerer Schaltfrequenz auszukommen.

Eine gewisse Schwierigkeit stellt die Montage des Drucksensors im Schädelknochen dar. Es ist zu diesem Zweck eine Adapterhülse bekannt, die ein Außengewinde aufweist und mit diesem unter gleichzeitigem Einschneiden des Gewindes in den Schädelknochen eingeschraubt wird. In dieser Adapterhülse wird dann der eigentliche Drucksensor wiederum eingeschraubt (GAAB, a.a.O., Abbildung 13, Bildteil Seite 9). Eine solche Adapterhülse hat vor allem zwei Nachteile : bei längerer Anwendung kann das Gewinde in den Schädelknochen einwachsen, so daß die Adapterhülse nach Beendigung der Untersuchung nur schwer zu entfernen ist. Zum anderen verkantet die Adapterhülse beim Einschrauben leicht, so daß die Membran des eigentlichen Drucksensors nicht mehr parallel zur Wandinnenfläche liegt, was auf jeden Fall bei der Messung des intrakraniellen Druckes zu beträchtlichen Meßfehlern führen kann. Die Adapterhülse, die als Treil des Sensorgehäuses angesehen werden kann, wird zur Vermeidung dieser Nachteile gemäß der Erfindung entsprechend Anspruch 9 ausgebildet.

Mit einer solchen Ausbildung wird sichergestellt, daß das Sensorgehäuse bei Implantation in den Schädelknochen nicht verkantet eingesetzt werden kann. Da das Sensorgehäuse mit der Adapterhülse ohne Verschraubung in den Schädelknochen eingesetzt wird, wird auch ein Einwachsen in den Schädelknochen zuverlässig verhindert. Aus diesem Grunde kann das Sensorgehäuse mit der Adapterhülse nach der Meßzeit leicht aus dem Schädelknochen entfernt werden.

Der in den Druckraum hineinragende Teil der Adapterhülse ist im unteren Bereich als Federhülse mit einem Wulst ausgebildet, der beim Einsetzen der Adapterhülse über den Innenrand der Bohrung hervorspringt und damit Fehlausfluchtungen der Adapterhülse mit der Wandbohrung korrigiert, bis die Hülsenschulter sich einwandfrei au der Bohrungsschulter abstützt. Zu diesem Zweck sollte der Wulst unter einem relativ steilen Winkel aus der Federhülsenaußenfläche hervorstehen. Die Rippen geben im letzten Teil des Einsetzweges eine gewisse Führung, vor allem aber beim Herausnehmen der Adapterhülse sorgen sie dafür, daß die einzelnen Federzungen allmählich nach innen gedrückt werden, so daß der Außenumfang verringert wird und auch ein relativ steil vorspringender Wulst auf einen ausreichend kleinen Außendurchmesser gebracht wird, um störungsfrei durch die engere Bohrung im Schädelknochen nach außen gezogen zu werden.

Wenn das Material, in das die Adapterhülse mit dem Drucksensor eingesetzt wird, vergleichweise weich ist, wie dies etwa beim Schädelknochen der Fall ist, kann die Führung der Adapterhülse beim Einsetzen und deren präzise axiale Ausfluchtung durch Abstützen auf der Bohrungsstufe erheblich verbessert werden, wenn zwischen der Schulter der Adapterhülse und der Bohrungsstufe gemäß Anspruch 10 ein Zwischenring angeordnet ist.

Die Erfindung ist in einem Ausführungsbeispiel anhand der Zeichnung näher erläutert. In der Zeichnung stellen dar :

Figur 1 einen Schnitt durch einen erfindungsgemäßen Drucksensor, implantiert in einen Schädelknochen ;

Figur 2 einen Längsschnitt durch eine Adapterhülse zur Montage eines Drucksensors und

Figur 3 einen Schnittlängs der Linie III-III in Figur 2.

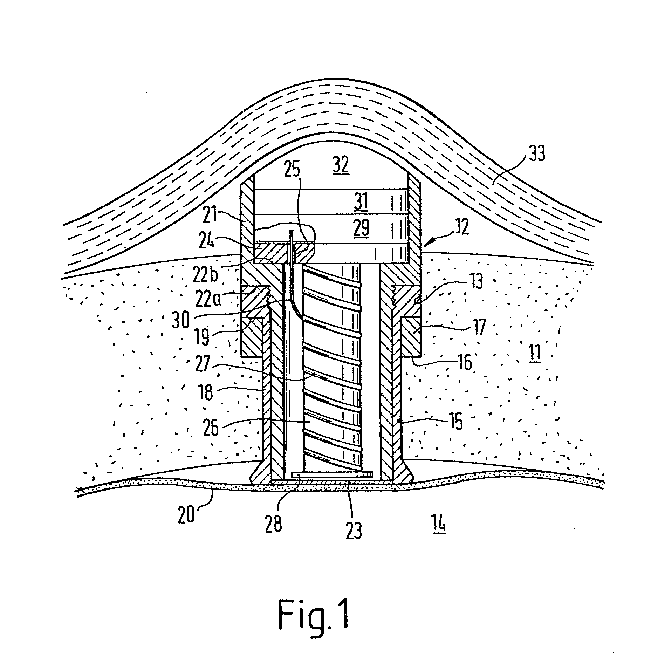

Gemäß Figur 1 ist in einem Schädelknochen 11 ein abgestuftes Bohrloch 12 vorgesehen, das einen weiteren, in Figur 1 oben dargestellten Teil 13 und einen engeren, dem Schädelinneren 14 zugewandten Teil 15 aufweist, die durch eine radial verlaufende Schulter 16 miteinander verbunden sind. Die Länge des engeren Bohrungsteils 15 ist fest vorgegeben, was in bekannter Weise durch Verwendung eines selbstauskuppelnden Minibohrers gewährleistet ist. Auf der Schulter 16 sitzt ein Zwischenring 17, dessen Innendurchmesser gleich dem Durchmesser des engsten Bohrungsteils 15·und dessen Außendurchmesser gleich dem Durchmesser des weiteren Bohrungsteils 13 ist und der aus einem inerten Werkstoff besteht, beispielsweise Edelstahl.

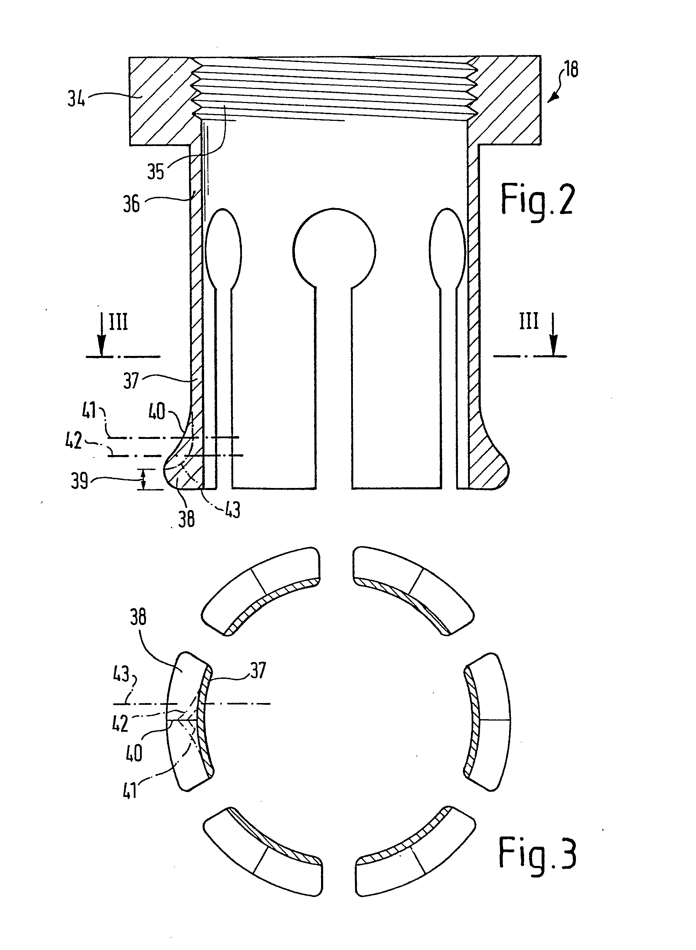

In die Bohrung und den Zwischenring 17 ist eine Adapterhülse 18 eingesetzt, die mit einer Radialschulter 19 auf dem Zwischenring 17 aufsitzt, sich durch den Zwischenring 17 und den engeren Bohrungsteil 15 hindurch um ein fest vorgegebenes Maß in den Raum zwischen Schädelknochen 11 und der das Gehirn umschließenden Dura 20 erstreckt. Die Adapterhülse 18 wird in Verbindung mit Figuren 2 und 3 später näher erläutert.

In die Adapterhülse wiederum ist ein erfindungsgemäßer Drucksensor eingesetzt, im dargestellten Ausführungsbeispiel eingeschraubt, es kommen jedoch auch andere Befestigungsarten, wie beispielsweise eine Bajonettmontage, in Frage. Zweckmäßigerweise ist diese Befestigung auf den Bereich oberhalb der Radialschultern 19 der Adapterhülse 18 beschränkt, um den dünnwandigen Teil der Hülse 18 von entsprechenden Belastungen freizuhalten.

Der Drucksensor selbst weist ein Gehäuse 21 in Form eines abgestuften zylindrischen Rohres auf, das auf der Innenseite elektrisch gut leitend ist ; vorzugsweise besteht das Rohr aus Edelstahl und ist innen mit Silber beschichtet. Der Außendurchmesser des weiteren Teils des Gehäuses 21 entspricht dem Durchmesser des weiteren Bohrungsteils 13, der übrige Teil des Gehäuses 21 ist an das Innere der Adapterhülse 18 angepaßt ; die von der Gehäusestufe gebildete Schulter 22a sitzt oben auf der Adapterhülse 18 auf und das der Dura 20 zugewandte Ende des Gehäuses schließt mit dem ebenfalls der Dura 20 zugewandten Ende der Adapterhülse 18 ab. Dieses der Dura 20 zugewandte Ende des Gehäuses 21 ist mit einer Membran 23 abgeschlossen, die in bekannter Weise elastisch nachgiebig ist und wenigstens auf der Innenseite elektrisch gut leitend ist. Sie kann aus einem Stück mit dem Gehäuse 21 gefertigt sein oder auch getrennt auf dieses aufgebracht sein, in letzterem Falle kommt als Werkstoff insbesondere Kupferberyllium in Frage. Auf der der Außenschulter 22a entsprechenden Innenschulter 22b des Gehäuses 21 ist ein Zwischenboden 24 angeordnet, der auf der Innenseite elektrisch gut leitend ist und eine Öffnung 25 aufweist. In dem durch das Innere des Gehäuses 21 und den Zwischenboden 24 definierten zylindrischen Topf oder Becher ist koaxial ein keramischer Spulenkörper 26 angeordnet, der eine aufgebrannte Silberwicklung 27 trägt sowie an seinem in der Zeichnung unten dargestellten Ende eine elektrisch leitende Beschichtung oder Platte 28. Die Silberwicklung 27 ist elektrisch gut leitend sowohl mit der Beschichtung oder Platte 28 als auch mit dem elektrisch leitenden Zwischenboden 24 verbunden. Die thermischen Ausdehnungskoeffizienten von Gehäuse 21 und Spulenkörper 26 sind bei der bevorzugten Ausführungsform gleich.

Auf der dem keramischen Isolierkörper 26 abgewandten Seite des Zwischen- oder Becherbodens 24 ist ein Oszillator 29, im dargestellten Ausführungsbeispiel eine FET-Schaltung, angeordnet, der über eine Leitung 30 elektrisch mit einem Punkt der Silberwicklung 27 verbunden ist, und zwar nacht Art eines Franklin-Oszillators, so daß der Einfluß des Oszillators auf den durch die Wicklung 27 und die Kapazität zwischen Platte 28 und Membran 23 gebildeten Schwingkreis (Topfkreis) klein gehalten wird, so daß der Effekt einer Alterung des Oszillators auch bei sehr großer Meßzeit (mehrere Monate oder sogar Jahre) praktisch vernachlässigt werden kann. Statt dieser galvanischen Ankopplung durch eine Leitung 30 kann auch eine bei Topfkreisen bekannte induktive Ankopplung vorgesehen sein, oder eine andere, den Schwingkreis nur wenig belastende Ankopplung.

Auf dem Oszillator 29 ist eine Stromversorgung 31 angeordnet. Wenn die Meßdauer relativ kurz ist (einige Tage) kann als Stromversorgung 31 eine Batterie in Form einer Knopfzelle vorgesehen sein ; ist aber auch mit größerer Meßdauer zu rechnen, wird zweckmäßigerweise eine bekannte drahtlose Stromversorgung verwendet, die im wesentlichen aus einer Empfangsantenne und einem Gleichrichter besteht.

Auf der Stromversorgung 31 befindet sich eine mit dem Oszillator 29 verbundene Antenne 32, mit das der Oszillatorsignal durch die das Ganze überdeckende Haut 33 abgestrahlt wird.

Bei einer Variante kann statt der schematisch als Spulenantenne dargestellten Antenne 32 ein Silikonschlauch vorgesehen sein, der durch die Haut 33 nach außen führt. Während bei der dargestellten Ausführungsform des Drucksensors der Innenraum des Gehäuses 21 unter bekanntem Druck steht, insbesondere einem definierten Unterdruck (Vakuum), könnte bei einer solchen Schlauchverbindung das Gehäuseinnere unter dem jeweils herrschenden Luftdruck stehen, so daß der Drucksensor ein Signal entsprechend der Differenz zwischen dem intrakraniellen Druck und dem Luftdruck abgibt, statt, wie beim dargestellten Ausführungsbeispiel, ein Signal entsprechend dem absoluten intrakraniellen Druck.

Statt überhaupt einer Antenne 32 ist bei einer anderen, im mechanischen Aufbau aber praktisch identischen, Ausführungsform eine infrarotes Licht emittierende Leuchtdiode vorgesehen ; in diesem Falle ist darauf zu achten, daß die verwendete Ausgußmasse infrarot-durchlässig ist. Ferner ist dann zweckmäßigerweise eine Frequenzteilerschaltung zwischen die Leuchtdiode und den Oszillator 29 geschaltet, so daß auch Leuchtdioden verwendet werden können, deren Schaltfrequenz unter der Grundfrequenz des erwähnten Schwingkreises liegt.

Der Drucksensor arbeitet wie folgt : Druckänderungen im Raum 14 werden durch die elastische Dura 20 auf die elastische Membran 23 weitergegeben, so daß deren Abstand von der Platte 28 und damit die Kapazität zwischen diesen Teilen ein Maß für den Druck im Raum 14 ist. Diese Kapazität bestimmt zusammen mit der Induktivität der Wicklung 27 die Frequenz des von diesen Teilen zusammen mit dem Gehäuse 21 und dem Zwischenboden 24 gebildeten Topfkreises, so daß der angeschlossene Oszillator 29 auf dieser Frequenz schwingt. Die Oszillatorschwingung wird als Oszillatorsignal von der Antenne 32 abgestrahlt und kann in bekannter Weise von einem getrennten Empfänger aufgenommen werden, an den ein Frequenzzähler oder dergleichen angeschlossen ist, dessen Ausgangssignal damit ein Maß für den Druck im Raum 14 darstellt.

Die Adapterhülse 18 ist in Figuren 2 und 3 näher dargestellt. Sie weist einen Ring 34 auf, in dessen Bereich ein Innengewinde 35 vorgesehen ist, das bei anderen Ausführungs formen durch eine andere Befestigungseinrichtung, wie erwähnt, ersetzt sein kann, und einen rohrförmigen Teil 36, der in eine Anzahl, im dargestellten Ausführungsbeispiel sechs, Federzungen wie 37 aufgespalten ist, so daß eine Federhülse gebildet wird. Der Außendurchmesser der Federhülse ist an den Innendurchmesser des engeren Bohrungsteils 15 angepaßt, mit Ausnahme des über die Innenbegrenzung der Bohrung 12 hervorstehenden Teils (Fig. 1), der einen größeren Außendurchmesser hat, so daß ein Außenwulst oder ein nach außen vorstehender Flansch 38 gebildet wird, dessen Axialabmessung 39 gleich dem Maß ist, um das die Hülse über die Innenfläche des Schädelknochens vorsteht (Fig. 1).

Ferner weist jede Federzunge 37 eine scharfkantige Rippe 40 auf, die vom Außenumfang des Wulstes 38 zur Außenfläche der zugehörigen Federzunge 37 an einer Stelle verläuft, die innerhalb des engeren Bohrungsteils 15 (Fig. 1) liegt; im dargestellten Ausführungsbeispiel erstreckt sich die Rippe 40 weiter in die Bohrung hinein als der Wulst 38 über den Außenumfang der Federhülse vorsteht. Die scharfkantige Rippe 40 geht von der scharfen Kante aus allmählich in die Außenfläche der Federhülse über, zur Veranschaulichung sind bei einer Federzunge in Fig. 3 zwei Schnitte in strichpunktierten Linien dargestellt, die die Außenkontur an zwei in Fig. 2 angegebenen Querschnitten 41, 42 angeben. Die sich daraus ergebende Außenwulstkontur ist in Fig. 2 als strichpunktierte Linie 43 entsprechend der in Fig. 3 eingetragenen Schnittlinie 43 angegeben.

Das Einsetzen eines Drucksensors erfolgt mit Hilfe dieser Adapterhülse in der Weise, daß nach dem Herstellen der abgestuften Bohrung 12, das in bekannter Weise erfolgt, der Zwischenring 17 zusammen mit der Adapterhülse 18 eingesetzt wird, wobei der Wulst in den Innenraum des Zwischenringes 17 eingeklemmt ist, so daß er auf den Innendurchmesser des engeren Bohrungsteils 15 zusammengedrückt ist. Sobald der Zwischenring 17 auf der Bohrungsschulter 16 aufliegt, wird die Adapterhülse 18 weitergeschoben, was ohne weiteres geht, weil der Außenumfang auch des Wulstes 38 auf das Maß des engeren Bohrungsteils 15 zusammengedrückt ist und deshalb ohne weiteres passieren kann. Sobald der Wulst, wie in Fig. 1 dargestellt, am unteren Ende aus der Bohrung 12, 15 austritt, können die einzelnen Federzungen nach außen ausweichen, so daß sich der Außenwulst 38 über die Unterseite des Schädelknochens 11 hinaus ausdehnen kann. Eventuelle Unregelmäßigkeiten in der Innenfläche werden dabei durch die schräg verlaufenden Rippen 40 ausgeglichen, die für eine gewisse Axialkraft sorgen, mit der der Ring 34 der Adapterhülse 18 auf den Zwischenring 17 gezogen wird, so daß die Adapterhülse 18 die gewünschte Lage einnimmt. Die Rippen 40 drücken sich dabei in den diesen gegenüber relativ weichen Knochen 11 ein. Anschließend wird der Drucksensor eingesetzt, also im dargestellten Ausführungsbeispiel eingeschraubt, und falls bis dahin die einzelnen Federzungen noch nicht die volle Ausdehnung erreicht haben, werden sie durch den Drucksensor auf die volle Ausdehnung nach außen gedrückt. Beim Entfernen des Drucksensors wird in umgekehrter Weise vorgegangen, d. h. der Drucksensor wird zunächst herausgenommen (herausgeschraubt), was ohne weiteres möglich ist, da sich die Adapterhülse 18 wegen der Rippen 40 nicht mitdrehen kann, und anschließend wird die Adapterhülse 18 herausgezogen, wozu anstelle des Drucksensors ein Ziehwerkzeug eingeschraubt werden kann, dabei nimmt der Außenwulst 38 den Zwischenring 17 mit. Dank der Rippen 40 die zu diesem Zweck in der dargestellten Weise unter einem relativ kleinen Winkel in die Außenfläche der jeweiligen Federzunge übergehen, werden die Federzungen 37 nach innen gedrückt, sobald die Adapterhülse 18 auch nur um ein geringfügiges Stück nach außen gezogen worden ist; durch die damit verbundene Keilwirkung ergibt sich auch eine ausreichende Kraft, die die Federzungen auf jeden Fall vom Knochen löst, auch wenn unvermeidbare Verklebungen erfolgt sind. Sofern der Durchmesser des weiteren Teils 13 der Bohrung 12 nicht größer ist als etwa 7 mm, wächst das Loch 12 nach der Entfernung des Sensors und der Adapterhülse 18 wieder zu. Da der Durchmesser des erfindungsgemäßen Drucksensors kleiner als 5 mm gehalten werden kann, kann auch der größte Durchmesser der Adapterhülse 18 klein genug gehalten werden, um ein Verwachsen des Bohrungsloches 12 zu ermöglichen.

**Patentansprüche**

1. Drucksensor, insbesondere zur Messung des intrakraniellen Druckes, mit einem Sensorgehäuse (21), in dem ein Topfschwingkreis mit einer festen Induktivität und einer in Abhängigkeit vom Druck veränderlichen kapazität sowie ein Oszillator (29) für den Topfschwingkreis mit seiner Stromversorgung (31) und ferner eine drahtlose Übertragungseinrichtung (32) für das Oszillatorsignal zu einer getrennten Empfängerbaueinheit aufgenommen sind, wobei der Topfschwingkreis einen zylindrischen Becher (21, 24) aus elektrisch leitendem Material, eine dessen Stirnfläche überspannende, elektrisch leitende elastische Membran (23) und einen koaxial im Becher (21, 24) angeordneten, in Abstand von der Membran (23) in einer flächigen Elektrode (28) endenden Innenleiter (27) als Induktivität aufweist, dadurch gekennzeichnet, daß der Topfschwingkreis als Wendeltopfkreis ausgebildet ist, dessen Innenleiter (27) wendelförmig auf eine koaxial im Becher (21, 24) angeordnete Keramik (26) aufgebracht ist, die an ihrer Stirnseite die flächige Elektrode (28) trägt, und daß der Oszillator (29) in dem Sensorgehäuse (21) auf der dem Innenleiter (27) abgewandten Rückseite des Becherbodens (24) gelegen ist.

2. Drucksensor nach Anspruch 1, dadurch gekennzeichnet, daß der wendelförmige Innenleiter (27) ein auf die Keramik (26) aufgebrannter Silberbelag ist.

3. Drucksensor nach einem der vorhergehenden Ansprüche, dadurch gekennzeichnet, daß die flächige Elektrode (28) einen größeren Durchmesser als die mit dem Innenleiter (27) versehene Keramik (26) aufweist und parallel zur Membran (23) angeordnet ist.

4. Drucksensor nach einem der vorhergehenden Ansprüche, dadurch gekennzeichnet, daß der Becher (21, 24) des Wendeltopfkreises aus einem Werkstoff besteht, der den gleichen Temperaturausdehnungskoeffizienten hat wie die Keramik (26), und daß die Innenfläche des Bechers mit einem elektrisch gut leitenden Material, insbesondere Silber, beschichtet ist.

5. Drucksensor nach Anspruch 4, dadurch gekennzeichnet, daß der Werkstoff des Bechers (21, 24) des Wendeltopfkreises Edelstahl ist.

6. Drucksensor nach einem der vorhergehenden Ansprüche, dadurch gekennzeichnet, daß der Oszillator (29) eine Schaltung zum Takten des Oszillators umfaßt.

7. Drucksensor nach Anspruch 1, dadurch gekennzeichnet, daß die Einrichtung zur Fernübertragung eine Antenne (32) umfaßt.

8. Drucksensor nach Anspruch 1, dadurch gekennzeichnet, daß die Einrichtung zur Fernübertragung einen an den Oszillator (29) angeschlossenen Frequenzteiler und eine diesem Frequenzteiler zugeordnete, im Infrarot arbeitende Leuchtdiode umfaßt.

9. Drucksensor nach einem der vorhergehenden Ansprüche, dadurch gekennzeichnet, daß zur Montage des Drucksensors in einer abgestuften, in einen Druckraum (14) hineinragenden Bohrung (12) das Sensorgehäuse (21) zumindest in einem Teil konzentrisch von einer Adapterhülse (18) umgeben ist, die eine sich auf der Bohrungsstufe (16) abstützende Schulter (19) aufweist und um ein fest vorgegebenes Maß in den Druckraum (14) hineinragt, daß der Teil der Adapterhülse (18) zwischen der Hülsenschulter (19) und dem dem Druckraum (14) zugewandten Ende als Federhülse (36) mit Federzungen (37) ausgebildet ist, daß der Außendurchmesser des in den Druckraum (14) hineinragenden Teils der Federhülse größer ist als der Innendurchmesser des engsten Teiles (15) der gestuften Bohrung (12), und daß die Federzungen (37) der Federhülse (36) jeweils eine scharfkantige Rippe (40) aufweisen, die sich jeweils vom Außenumfang der Federhülse (36) zu einer sich innerhalb des engsten Teiles (15) der gestuften Bohrung (12) liegenden Stelle erstrecken.

10. Drucksensor nach Anspruch 9, dadurch gekennzeichnet, daß zwischen der Schulter (19) der Adapterhülse (18) und der Bohrungsstufe (16) ein Zwischenring (17) angeordnet ist.

## Claims

1. A pressure gauge, particularly for measuring intracranial pressure, comprising a gauge housing (21) accommodating therein a pot oscillating circuit having a fixed inductance and a capacitance which is variable in response to pressure as well as an oscillator (29) for the pot oscillating circuit with its power supply (31) as well as a wireless transmission means (32) for transmitting the oscillator signal to a separate receiver unit, said pot oscillating circuit including a cylindrical cup (21, 24) of electrically conductive material, an electrically conductive, resilient diaphragm (23) which spans the end face of said cup and, as an inductor, an inner conductor (27) which is arranged within the cup (21, 24) such that it is coaxial therewith and which ends in a plane electrode (28) in spaced relation-ship with the diaphragm (23), characterized in that the pot oscillating circuit is designed as a helix pot circuit whose inner conductor (27) is applied in the form of a helix to ceramics (26) arranged within the cup (21, 24) such that they are coaxial therewith and carrying at the end face thereof the plane electrode (28), and that the oscillator (29) is located in said gauge housing (21) at the back of the cup bottom (24) facing away from the inner conductor (27).

2. A pressure gauge according to claim 1, characterized in that the helical inner conductor (27) is a silver coating which has been applied to the ceramics (26) by firing on.

3. A pressure gauge according to one of the preceding claims, characterized in that the plane electrode (28) has a larger diameter than the ceramics (26) provided with the inner conductor (27) and that said plane electrode is arranged parallel to the diaphragm (23).

4. A pressure gauge according to one of the preceding claims, characterized in that the cup (21, 24) of the helix pot circuit consists of a material having the same coefficient of thermal expansion as the ceramics (26), and that the inner surface of the cup is coated with an electrically highly conductive material, in particular with silver.

5. A pressure gauge according to claim 4, characterized in that the material of the cup (21, 24) of the helix pot circuit is high-quality steel.

6. A pressure gauge according to one of the preceding claims, characterized in that the oscillator (29) includes a circuit by means of which said oscillator is clocked.

7. A pressure gauge according to claim 1, characterized in that the means for remote transmission includes an aerial (32).

8. A pressure gauge according to claim 1, characterized in that the means for remote transmission includes a frequency divider connected to the oscillator (29) as well as a light-emitting diode associated with said frequency divider and operating in the infrared region.

9. A pressure gauge according to one of the preceding claims, characterized in that, for mounting the pressure gauge in a stepped hole (12) projecting into a pressure chamber (14), the gauge housing (21) is, at least along a part thereof, concentrically surrounded by an adapter sleeve (18) which is provided with a shoulder (19) resting on the step (16) of said hole and which projects into said pressure chamber (14) by a fixedly predetermined length, that the adapter sleeve part located between the sleeve shoulder (19) and the end facing the pressure chamber (14) is constructed as a spring sleeve (36) with spring tongues (37), that the outer diameter of the spring sleeve part projecting into the pressure chamber (14) is larger than the inner diameter of the narrowest part (15) of the stepped hole (12), and that each of the spring tongues (37) of the spring sleeve (36) is provided with a sharpedged rib (40), said ribs extending from the respective outer circumference of the spring sleeve (36) to a point located within the narrowest part (15) of the stepped hole (12).

10. A pressure gauge according to claim 9, characterized in that an intermediate ring (17) is arranged between the shoulder (19) of the adapter sleeve (18) and the step (16) of said hole.


**Revendications**

1. Capteur de pression, en particulier pour mesurer la pression intra-crânienne, comprenant un boitier (21) de capteur logeant un circuit oscillant à cavité présentant une inductance fixe et une capacité variable en fonction de la pression, ainsi qu'un oscillateur (29) pour le circuit oscillant à cavité, muni d'une alimentation en courant (31) et, en outre, d'un dispositif de transmission sans fil (32) du signal délivré par l'oscillateur vers une unité de réception séparée, le circuit oscillant à cavité présentant un boitier cylindrique (21, 24) en matériau conducteur d'électricité, une membrane élastique (23) conductrice d'électricité, tendue sur la surface frontale dudit boitier, et un conducteur intérieur (27) formant l'inductance, disposé de façon coaxiale dans le boitier (21, 24) et se terminant par une électrode plate (28) espacée de la membrane (23), caractérisé en ce que le circuit oscillant à cavité est formé d'une cavité hélicoïdale dont le conducteur intérieur (27) est enroulé en hélice autour d'une céramique (26) disposée de façon coaxiale dans le boitier (21, 24), la face frontale de cette dernière supportant l'électrode plate (28), et en ce que l'oscillateur (29) disposé dans le boitier (21) du capteur, est placé sur la face arrière du fond (24) du boitier, opposée au conducteur intérieur (27).

2. Capteur de pression selon la revendication 1, caractérisé en ce que le conducteur intérieur (27) hélicoïdal est formé par une couche d'argent cuite sur la céramique (26).

3. Capteur de pression selon l'une des revendications précédentes, caractérisé en ce que l'électrode plate (28) présente un diamètre plus grand que celui de la céramique (26) pourvue du conducteur intérieur (27) et est disposée parallèlement à la membrane (23).

4. Capteur de pression selon l'une des revendications précédentes, caractérisé en ce que le boitier (21, 24) de la cavité hélicoïdale est réalisé en matériau présentant le même coefficient de dilatation thermique que celui de la céramique (26) et en ce que la surface intérieure du boitier est recouverte d'un matériau bon conducteur de l'électricité, en particulier de l'argent.

5. Capteur de pression selon la revendication 4, caractérisé en ce que le matériau constituant le boitier (21, 24) de la cavité hélicoïdale est de l'acier allié.

6. Capteur de pression selon l'une des revendications précédentes, caractérisé en ce que l'oscillateur (29) comprend un circuit de synchronisation de l'oscillateur.

7. Capteur de pression selon la revendication 1, caractérisé en ce que le dispositif de transmission à distance comprend une antenne (32).

8. Capteur de pression selon la revendication 1, caractérisé en ce que le dispositif de transmission à distance comprend un diviseur de fréquence raccordé à l'oscillateur (29) et une diode luminescente travaillant dans l'infra-rouge, associée à ce diviseur de fréquence.

9. Capteur de pression selon l'une des revendications précédentes, caractérisé en ce que pour monter le capteur de pression dans un alésage étagé (12), débouchant dans une chambre de pression (14), le boitier (21) du capteur, au moins dans une partie concentrique, est entouré d'un manchon d'adaptation (18) qui présente un épaulement (19) s'appuyant sur le gradin (16) de l'alésage et qui fait saillie dans la chambre de pression (14), suivant une dimension prédéterminée fixe, en ce que la partie du manchon d'adaptation (18) comprise entre l'épaulement (19) du manchon et l'extrémité adjacente à la chambre de pression (14), est constituée d'un manchon élastique (36) présentant des languettes élastiques (37), en ce que le diamètre extérieur de la partie du manchon élastique faisant saillie dans la chambre de pression (14) est plus grand que le diamètre intérieur de la partie la plus étroite (15) de l'alésage étagé (12), et en ce que chacune des languettes élastiques (37) du manchon élastique (36) présente une nervure (40) à angles vifs qui s'étend de la périphérie extérieure du manchon élastique (36) vers un endroit situé dans la partie la plus étroite (15) de l'alésage étagé (12).

10. Capteur de pression selon la revendication 9, caractérisé en ce qu'une bague (17) est interposée entre l'épaulement (19) du manchon d'adaptation (18) et le gradin (16) de l'alésage.

Fig.1

Fig.2

Fig.3